# EUROPÄISCHE PATENTSCHRIFT

(11) **EP 0 925 028 B1**
(45) Veröffentlichungstag und Bekanntmachung des Hinweises auf die Patenterteilung: **19.09.2001**
(21) Anmeldenummer: 98928316.3
(22) Anmeldetag: 25.05.1998
(51) Int. Cl.: A61B 17/00, A61B 17/28

(54) **ZERLEGBARES MEDIZINISCHES INSTRUMENT MIT SELBSTORIENTIERENDER KUPPLUNG**
DISMOUNTABLE MEDICAL INSTRUMENT WITH A SELF-ORIENTING COUPLING
INSTRUMENT MEDICAL DEMONTABLE AVEC ACCOUPLEMENT A ORIENTATION AUTOMATIQUE

(30) Priorität: 27.05.1997 DE 19722062
(43) Veröffentlichungstag der Anmeldung: 30.06.1999
(73) Patentinhaber: Karl Storz GmbH & Co. KG, 78532 Tuttlingen (DE)
(72) Erfinder: DITTRICH, Horst, D-78194 Immendingen (DE); BACHER, Uwe, D-78532 Tuttlingen (DE); RUDISCHHAUSER, Jürgen, D-78532 Tuttlingen (DE); IRION, Klaus, M., D-78576 Liptingen (DE)
(74) Vertreter: Weller, Wolfgang, Dr.rer.nat.
(86) Internationale Anmeldenummer: EP9803065
(87) Internationale Veröffentlichungsnummer: WO9853743

(56) Entgegenhaltungen:
- DE-U- 9 408 931
- US-A- 5 293 878
- US-A- 5 507 297

## Beschreibung

Die Erfindung betrifft ein medizinisches Instrument, das in mehrere Bauteile, insbesondere in einen Handgriff und in einen Rohrschaft zerlegbar ist, wobei ein Bauteil, insbesondere der Rohrschaft gegenüber einem weiteren Bauteil, insbesondere dem Handgriff mittels eines an dem weiteren Bauteil angeordneten Drehelements verdrehbar ist, mit einer Kupplung zum lösbaren, drehschlüssigen Verbinden des einen Bauteiles mit dem Drehelement des weiteren Bauteiles, wobei die Kupplung zwei längs einer Kupplungsachse ineinandergreifende Kupplungshälften aufweist, wobei auf jeder Kupplungshälfte im radialen Abstand zur Kupplungsachse zumindest zwei Zähne angeordnet sind, wobei sich an jedem Zahn in Umfangsrichtung zumindest eine Lücke anschließt, in die passend ein Zahn der anderen Kupplungshälfte eintreten kann.

Ein derartiges Instrument ist aus der US-A-5 507 297 bekannt.

Aus der US-A-5 293 878 ist es bekannt, die Zähne in Umfangsrichtung mit zumindest einer Abschrägung zu versehen.

In mehrere Bauteile zerlegbare medizinische Instrumente finden in der Chirurgie breite Verwendung. Für minimal-invasive Eingriffe geeignete Instrumente sind als Rohrschaftinstrumente ausgebildet, die einen proximalen Handgriff und einen Rohrschaft aufweisen. Durch den Rohrschaft wird ein Arbeitseinsatz geführt, der an seinem distalen Ende bspw. Maulteile mit Scheren- oder Zangenfunktion, Spatel, Nadeln, Elektroden, Schlingen und dgl. aufweist.

Das Zerlegen der Instrumente in Handgriff, Rohrschaft und Arbeitseinsatz dient dabei einer optimalen Reinigung und erlaubt einen flexiblen Einsatz der Instrumente durch Kombination verschiedener Bauteile miteinander.

Das Verbinden der Bauteile muß jedoch einfach und schnell gehen, um ein Austauschen der Bauteile, unter Umständen auch während der Operation, zu erlauben, ohne den reibungslosen Ablauf der Operation zu stören.

Eine Schwierigkeit beim Verbinden der Bauteile, meist eines Handgriffs mit einem den Arbeitseinsatz enthaltenden Rohrschaft, entsteht dadurch, daß es wünschenswert ist, den Rohrschaft um dessen Längsachse verdrehbar mit dem Handgriff zu verbinden. Dann können Maulteile nämlich in jede beliebige Drehstellung zum Handgriff gebracht werden, so daß der Operateur die günstigste Relativdrehstellung zwischen Handgriff und Maulteilen einstellen kann.

Durch das Vorsehen von Zähnen mit einer Abschrägung werden die Kupplungshälften während des axialen Zusammenfügens und Aufeinandertreffens in jeder beliebigen Stellung so abgelenkt, daß die Zähne aneinander vorbeigleiten und unwillkürlich in benachbarte Lücken eingeführt werden.

Es ist daher Aufgabe der vorliegenden Erfindung, ein in mehrere Bauteile zerlegbares medizinisches Instrument zu schaffen, bei dem das Verbinden der Bauteile schnell und einfach und ohne hohe Aufmerksamkeit sicher durchzuführen ist.

Erfindungsgemäß wird die Aufgabe bei einem Instrument der eingangs genannten Art dadurch gelöst, daß jeder Zahn in Umfangsrichtung mit zumindest einer Abschrägung versehen ist, und daß die Zähne der einen Kupplungshälfte umfänglich über Materialbrücken miteinander verbunden sind, und daß die Materialbrücken radial zurückgesetzte Aussparungen aufweisen, in die Zähne der anderen Kupplungshälfte derart einrücken, daß die beiden Kupplungshälften gegen axiales Abziehen gesperrt sind.

Diese Maßnahme hat den Vorteil, daß durch die Abschrägung nicht nur die Zähne der einen Kupplung in die entsprechenden Lücken der anderen Kupplungshälfte einrücken, sondern daß zugleich ein Zahn der einen Kupplungshälfte in die radial zurückgesetzte Aussparung der anderen Hälfte einrückt, wodurch ein axiales Trennen der Kupplung gesperrt ist.

In einer Ausgestaltung der Erfindung ist jeder Zahn beidseitig einer Spitze mit einer Abschrägung versehen.

Diese Maßnahme hat den Vorteil, daß je nach Auf treffen der Zähne aufeinander in die eine oder in die entgegengesetzte Umfangsrichtung abgelenkt wird und schon nach relativ kurzer Drehbewegung die Zähne ausgerichtet in die Lücken eintreten können. Bei Zähnen mit nur einer Abschrägung erfolgt eine Ablenkung immer nur in eine Umfangsrichtung.

In einer weiteren Ausgestaltung sind auf jeder Kupplungshälfte jeweils zwei Zähne vorgesehen.

Diese Maßnahme hat den Vorteil, ein sicheres Ineinandergreifen der Kupplungshälften mit relativ geringem Drehwinkel zu gewährleisten.

In einer besonders bevorzugten Ausführung sind auf einer Kupplungshälfte zwei Zähne und auf der zweiten Kupplungs- hälfte vier Zähne vorgesehen.

Dies hat den Vorteil, daß sichergestellt ist, daß auf jeden Fall zwei Zähne aufeinandertreffen und daher ein sicheres Ineinandergreifen ermöglicht ist. Dabei wird die Beanspruchung der vier Zähne gering gehalten, so daß sich die vier Zähne weniger schnell abnutzen und die Lebensdauer der Kupplung erhöht wird. Dadurch ist möglich, die vier Zähne auf der Instrumentenhälfte anzuordnen, die wertvoller ist, oder eine längere Standzeit haben soll.

In einer weiteren Ausführung stehen die Abschrägungen der Zähne in einem spitzen Winkel zueinander.

Diese Ausgestaltung bietet den Vorteil, aufgrund der relativ stark geneigten Auflaufflächen eine sichere Ineinanderführung der Kupplungshälften bei relativ langem axialen Kupplungsweg mit relativ geringer Relativverdrehung der Kupplungshälften zu gewährleisten.

Bei einem Zahn mit nur einer Abschrägung liegt die Spitze etwa in der Verlängerung einer Zahnflanke und bildet damit den spitzen Winkel.

In einer weiteren Ausgestaltung stehen die Abschrägungen der Zähne in einem stumpfen Winkel zueinander.

Diese Maßnahme erlaubt Zähne mit geringer Bauhöhe.

In einer besonders bevorzugten Ausgestaltung stehen die Abschrägungen der Zähne in einem Winkel von etwa 90° zueinander.

Diese Geometrie bietet den Vorteil, bei geringer Bauhöhe der Zähne zugleich eine sichere Ineinanderführung der Kupplungshälften zu ermöglichen.

In einer weiteren bevorzugten Ausgestaltung weisen die Spitzen der Zähne scharfe Kanten auf.

Hierbei ist vorteilhaft, daß auch bei diametralem Aufeinandertreffen von zwei Spitzen ein sofortiges Abgleiten in die eine oder andere Richtung und damit ein Einführen der Zähne in die entsprechenden Lücken gewährleistet ist.

In einer weiteren Ausführung sind die Spitzen der Zähne abgerundet.

Diese Maßnahme hat den Vorteil, daß die Zähne widerstandsfähiger gegen Abnutzungserscheinungen bei direktem Aufeinandertreffen der Spitzen sind.

In einer weiteren Ausgestaltung der Erfindung verlaufen die Abschrägungen der Zähne nach außen gekrümmt.

Diese Maßnahme hat den Vorteil, daß zwei an deren Abschrägungen aufeinandertreffende Zähne beim Ineinanderstecken der Kupplungshälften bei der durch das Aufeinandertreffen der Zähne verursachten Drehbewegung im wesentlichen nur punktförmig aneinander anliegen, somit der Drehvorgang sehr reibungsarm abläuft.

In einer weiteren Ausgestaltung der Erfindung weisen die Zähne magnetische Spitzen auf, wobei die magnetischen Spitzen der einen Kupplungshälfte gegenpolig zu den magnetischen Spitzen der anderen Kupplungshälfte sind.

Diese Maßnahme hat nun den Vorteil, daß aufgrund der Gegenpoligkeit der Spitzen diese, sollten sie einmal genau aufeinander zu gerichtet sein, aufgrund der Abstoßungskräfte schon vor dem Zusammentreffen für eine Ablenkung in die eine oder andere Richtung sorgen, somit besonders sicher und besonders sanft der Selbstorientierungs- bzw. Selbstausrichtungsvorgang abläuft, was insbesondere bei sehr schlanken Instrumenten zur Materialschonung beiträgt.

In einer bevorzugten Ausgestaltung werden die beiden Bauteile über eine von der Kupplung getrennte Verriegelung gegen axiales Trennen verriegelt.

Hierbei ist von Vorteil, daß die Verriegelung an einer beliebigen Stelle innerhalb des Bereichs, in dem die beiden Bauteile ineinandergreifen, angeordnet sein kann. Außerdem ist hierbei eine besonders einfache Ausgestaltung, bspw. in Form einer Rastnut auf einem Bauteil und einer Rastnase auf dem anderen Bauteil, möglich.

In einer weiteren Ausgestaltung der Erfindung sind bei einem Rohrschaftinstrument vier Zähne an der Außenseite des Rohrschaftes ausgebildet und, axial davon beabstandet, ist eine Ringnut der Verriegelung ausgespart, und an der Innenseite des Drehelementes am Handgriff sind zwei diametral gegenüberliegende Zähne vorgesehen, wobei am Handgriff ein Riegel vorgesehen ist, der sperrend in die Ringnut eingreift.

Die vier Zähne an der Außenseite des Rohrschaftes sind an Stellen vorgesehen, die für eine Bearbeitung günstig liegen, es können die Zähne aus einem Vollmaterial herausgearbeitet werden. Gleichermaßen einfach ist die Ringnut in die Außenseite des Rohrschaftes einzubringen.

An der Innenseite des Drehelementes müssen lediglich die beiden diametral gegenüberliegenden Zähne ausgespart werden. Am Handgriff kann in bedienungsfreundlicher Anordnung der Riegel vorgesehen sein, der in die Ringnut eingreift, um ein axiales Sperren zu bewerkstelligen. Dies ist fertigungstechnisch einfach und handhabungsfreundlich.

Die Erfindung wird nachfolgend anhand eines ausgewählten Ausführungsbeispiels in Zusammenhang mit den beiliegenden Zeichnungen näher beschrieben und erläutert. Es zeigen:
- Fig. 1: eine perspektivische Explosionsansicht eines zerlegbaren medizinischen Instrumentes,
- Fig. 2: eine stark vergrößerte ausschnittsweise Darstellung der Bauelemente, die die beiden Kupplungshälften der Kupplung tragen, und
- Fig. 3: einen Querschnitt durch ein Kupplungselement bei geschlossener Kupplung.

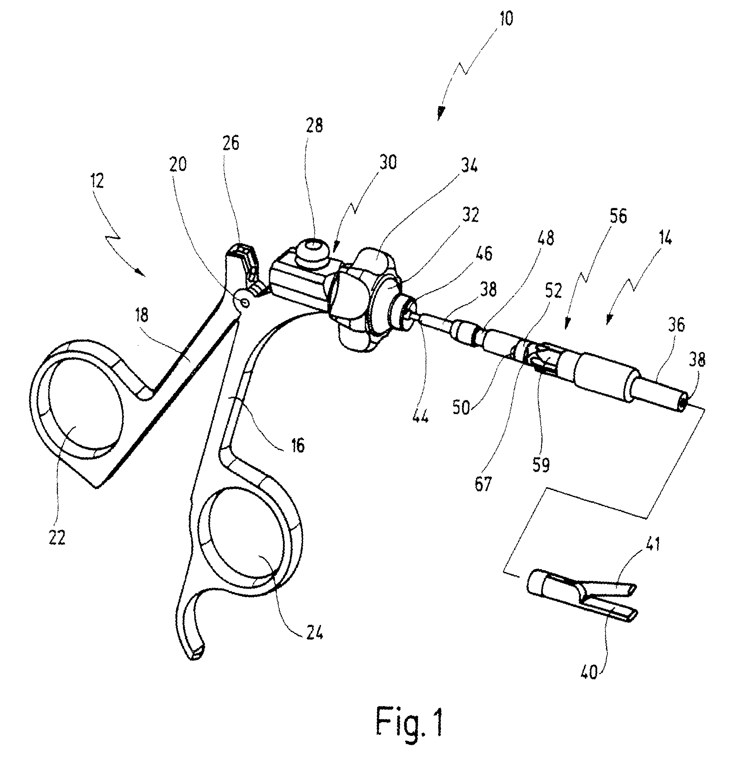

Ein in den Fig. 1 bis 3 gezeigtes zerlegbares medizinisches Instrument ist in seiner Gesamtheit mit der Bezugsziffer 10 versehen.

Das Instrument 10 weist als ein Bauteil einen Handgriff 12 und als weiteres Bauteil einen lösbar damit verbindbaren Rohrschaft 14 auf.

Der Handgriff 12 weist ein festes Griffelement 16 auf, an dem ein bewegliches Griffelement 18 über ein Scharnier 20 verschwenkbar angebracht ist.

Beide Griffelementen 16 und 18 weisen am äußeren Ende eine Fingeröse 22 bzw. 24 auf.

Am oberen Ende des beweglichen Griffelementes 18 ist eine Pfanne 26 ausgespart, deren Sinn und Zweck später beschrieben wird.

Am oberen Ende des festen Griffelementes 16 ist ein Riegelknopf 28 einer Verriegelung 30 angeordnet.

Am distalen, dem proximalen Ende des Rohrschaftes 14 zugewandten Ende des festen Griffelementes 16 ist ein Drehelement 32 in Form eines Stellrades 34 angeordnet. Das Stellrad 34 ist drehbar angebracht.

Der Rohrschaft 14 weist ein Rohr 36 auf, in dessen Inneren ein stabförmiges Betätigungselement 38 aufgenommen ist.

Distal überragt das Betätigungselement 38 das Rohr 36 und trägt zwei Maulteile 40 und 42.

Das Betätigungselement 38 überragt proximal in einem Abschnitt 44 das Rohr 36 und trägt an seinem äußeren proximalen Ende eine Kugel 46.

Die Kugel 46 ist dazu vorgesehen, in der Pfanne 26 des beweglichen Griffelementes 18 aufgenommen zu werden.

Dazu wird, wie das in Fig. 1 angedeutet ist, der Rohrschaft 14 samt Betätigungselement 38 durch eine durchgehende Öffnung im festen Griffelement 16, die auch mittig durch das Stellrad 34 hindurchreicht, durchgeschoben, bis die Kugel 46 in der Pfanne 26 zum Liegen kommt. Dies kann in einer bestimmten Schwenkstellung des beweglichen Griffelementes 18 erfolgen. Wird aus dieser Stellung das bewegliche Griffelement 18 bewegt, wird die Schwenkbewegung über das Kugelpfannengelenk in eine lineare Verschiebebewegung des Betätigungselementes 38 umgesetzt, das wiederum durch diese Linearbewegung die Maulteile 40, 42 öffnet bzw. schließt.

An der Außenseite des proximalen Endes des Rohres 36 ist eine erste Kupplungshälfte 56 angeordnet.

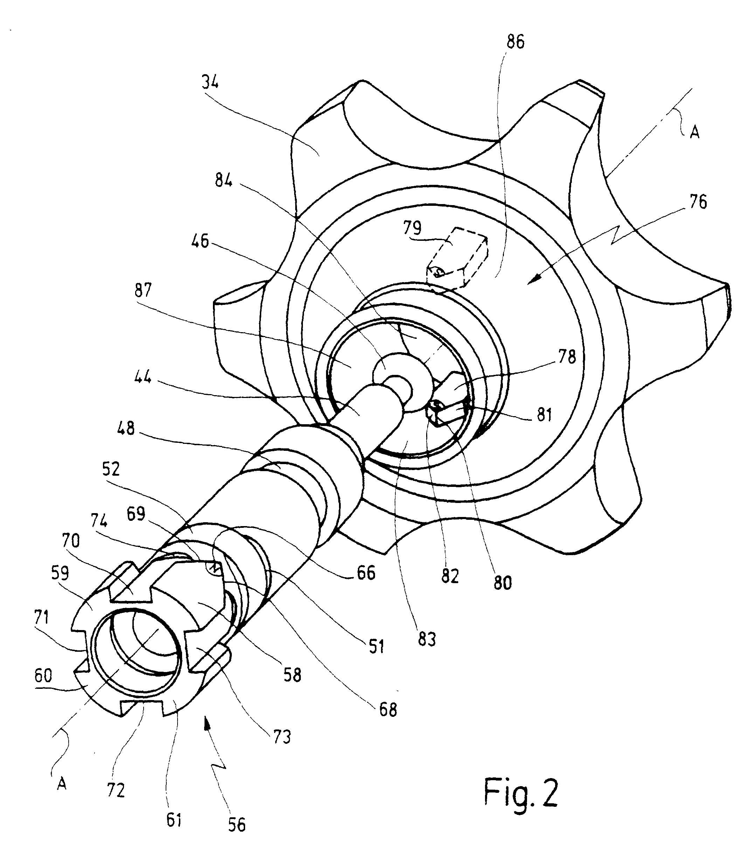

Die Kupplungshälfte 56 weist, wie das insbesondere aus der perspektivischen Ansicht von Fig. 2 zu ersehen ist, vier umfänglich jeweils um 90° versetzte Zähne 58, 59, 60, 61 auf. An jeden Zahn schließt sich in umfänglicher Richtung jeweils eine Lücke 62, 63, 64, 65 an. Die umfängliche Breite eines jeden Zahnes 58, 59, 60, 61 ist gleich wie die umfängliche Breite einer Lücke 62, 63, 64, 65.

Jeder Zahn weist eine dem Handgriff 12 zugewandte Spitze auf, wobei in Fig. 2 die Spitze 66 des Zahnes 58 ersichtlich ist, in Fig. 1 die Spitze 67 des Zahnes 59.

Beidseits jeder Spitze eines Zahnes ist eine Abschrägung vorhanden, in Fig. 2 sind die Abschrägungen 68 und 69 des Zahnes 58 beidseits der Spitze 66 ersichtlich. Die Abschrägungen erstrecken sich somit in umfänglicher Richtung und sind, von einer Radialebene auf Höhe der Spitze 66 aus gesehen, vom proximalen zum distalen Ende des Schaftes 14 hin, geneigt. Die Abschrägungen schließen einen Winkel von ca. 90° ein.

Wie aus der perspektivischen Darstellung von Fig. 2 zu ersehen, sind die vier Zähne 58, 59, 60 und 61 in umfänglicher Richtung über hier nicht näher bezeichnete Materialbrücken verbunden, in denen ebene, radial etwas zurückgesetzte Aussparungen 70, 71, 72 und 73 vorhanden sind.

Somit geht die kreisrunde Außenkontur des Schaftes bzw. des Rohres 36 jeweils über eine Rampe, in Fig. 2 ist die Rampe 74 der Aussparung 70 ersichtlich, in eine Aussparung über.

Eine zweite Kupplungshälfte 76 ist am Stellrad 34 des festen Griffelementes 16 angeordnet.

Die zweite Kupplungshälfte 76 weist zwei diametral gegenüberliegende Zähne 78 und 79 auf, die jeweils eine Spitze aufweisen, in der Fig. 2 ist die Spitze 80 des Zahnes 78 ersichtlich.

Jede Spitze ist beiseits mit einer Abschrägung 81 bzw. 82 versehen.

Die beiden Zähne 78 und 79 sind an einer Innenseite 83 einer mittigen durchgehenden Öffnung 84 im Stellrad 34 angeordnet.

Die Kontur der Zähne 78 und 79 bezüglich der Spitze und der Abschrägungen 81 und 82 ist identisch wie die Kontur der Zähne 58, 59, 60 und 61 der ersten Kupplungshälfte 56. Allerdings stehen die Spitzen 80 der Zähne 78, 79 den Spitzen der Zähne 58, 59, 60, 61 gegenüber. In umfänglicher Richtung gesehen bestehen zwischen den beiden diametral gegenüberstehenden Zähnen 78 und 79 Lücken 86 und 87.

Wie dies insbesondere aus Fig. 2 zu entnehmen ist, sind die Zähne 58 bis 61 der ersten Kupplungshälfte 56 im Bereich ihrer Spitzen 63 bis 66 mit einem Magneteinsatz versehen (Symbol N).

Dementsprechend sind die Zähne 78 und 79 der zweiten Kupplungshälfte 76 im Bereich ihrer Spitzen ebenfalls mit Magneteinsätzen, jedoch gegenpolig zu denen der Zähne der Kupplungshälfte 56 versehen (Symbol S).

Beide Kupplungshälften 56 und 76 bilden insgesamt gesehen eine Kupplung 90.

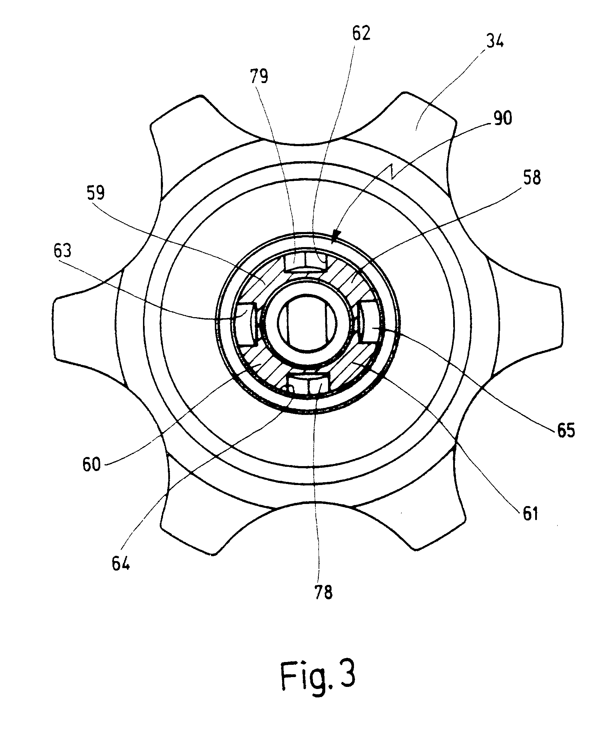

Zum Schließen der Kupplung 90 wird der Rohrschaft 14, wie das in den Fig. 1 und 2 dargestellt ist, in die zentrale Öffnung 84 des Stellrades 34 bzw. des Handgriffes 12 eingeschoben. Treffen die beiden Zähne 78 und 79 der zweiten Kupplungshälfte 76 genau auf zwei diametral gegenüberliegende Lücken 62 und 64, wie das in Fig. 3 dargestellt ist, können die beiden Bauelemente Rohrschaft 14 und Handgriff 12 ohne Relativverdrehung ineinandergeschoben werden. Dies ist exakt nur in zwei um 90° zueinander versetzten Relativstellungen möglich.

In allen anderen Relativdrehstellungen treffen die Abschrägungen 81, 82 der Zähne 78 und 79 auf entsprechende Abschrägungen von zwei der vier Zähne 58, 59, 60 und 61, wodurch dann eine zwangsgeführte Relativverdrehung der beiden Elemente um die Kupplungsachse A erfolgt, bis eine solche Ausrichtung vorliegt, daß die beiden diametral gegenüberstehenden Zähne 78, 79 in entsprechend diametral gegenüberliegende Lücken in der ersten Kupplungshälfte 56 eintreten können. Die Kupplung 90 ist somit selbstorientierend bzw. selbstausrichtend.

Die Einleitung der Verdrehbewegung beim Annähern der Zähne wird noch durch die Abstoßungskräfte der gegenpoligen magnetischen Spitzen unterstützt. Sollte die Kupplung einmal so angesetzt werden, daß die Spitze 66 des Zahnes 58 exakt auf die Spitze des Zahnes 79 trifft, sorgt die Abstoßungskraft der Pole schon vor dem mechanischem Aufeinandertreten der beiden Spitzen für eine Verdrehung im oder gegen den Uhrzeigersinn um die Kupplungsachse A, so daß ausgeschlossen ist, daß die Spitzen direkt aufeinandertreffen. Die Abstoßungskräfte unterstützen auch dann die weitere Drehbewegung der einmal eingeschlagenen Drehrichtung.

Die Anordnung der beiden Zähne 78, 79 ist dabei so, daß sie mit sanftem radialem Druck über die Außenseite des Schaftes 14 gleiten und dann über die Rampen, gezeigt ist lediglich die Rampe 74 in Fig. 2, in die etwas radial zurückgesetzte Aussparung 70 bzw. 72 einrücken.

Durch die Rampe 74 bzw. dann die entsprechend gegenüberliegende Rampe ist schon ein gewisses Verriegeln der beiden Zähne 78 und 79 gegenüber axialem Abziehen gewährleistet.

Das eigentliche Verriegeln gegen axiales Abziehen erfolgt über die Verriegelung 30, d.h. der Verriegelungsknopf 28 greift in die Ringnut 48 ein.

Sind die Zähne 78 und 79 der einen Kupplungshälfte 76 in die entsprechenden Lücken der anderen Kupplungshälfte 56 eingerückt, ist eine formschlüssig und somit auch eine drehschlüssige Verbindung zwischen dem Stellrad 34 und dem Rohrschaft 14 geschaffen.

Im montierten Zustand kann somit über das Stellrad 34 der Rohrschaft 14 samt darin aufgenommenem Betätigungselement 38 um die Kupplungsachse A relativ zum Handgriff 12 verdreht werden, wodurch die Maulteile 40 und 42 in eine für den Operateur günstige relative Drehstellung zum Handgriff 12 gebracht werden können. Durch die Anordnung des Kugelpfannengelenkes ist diese Drehbarkeit in jeder Schwenkstellung des beweglichen Griffelementes 18 möglich.

Damit keine Relativdrehung zwischen dem Betätigungselement 38 und dem Rohrschaft 14 stattfindet, ist das Betätigungselement 38 in dem Bereich zweier Klemmstücke 50, 51 abgeflacht. Die Klemmstücke 50, 51 greifen in die Abflachung ein und werden durch einen Haltering 52 gehalten. Dadurch ist dann ein axiales Verschieben des Betätigungselementes 38 im Rohr 36 möglich, ein Verdrehen des Betätigungselementes 38 relativ zum Rohr 36 ist aber nicht möglich.

Zum Lösen der Kupplung 90 muß der Riegelknopf 28 betätigt werden, so daß dessen hier nicht gezeigtes Riegelelement aus der Ringnut 48 austritt, anschließend kann der Rohrschaft 14 abgezogen werden, wobei eine gewisse Kraft notwendig ist, um die beiden Zähne 78 und 79 über die Rampen 74 der entsprechenden Aussparungen hinweg zu bewegen.

Dadurch ist sichergestellt, daß bei unachtsamer Handhabbarkeit bei gelöstem Riegelknopf 28 der Rohrschaft 14 nicht vom Handgriff 12 abfällt.

## Patentansprüche

1. Medizinisches Instrument, das in mehrere Bauteile, insbesondere in einen Handgriff (12) und in einen Rohrschaft (14) zerlegbar ist, wobei ein Bauteil, insbesondere der Rohrschaft (14) gegenüber einem weiteren Bauteil, insbesondere dem Handgriff (12) mittels eines an dem weiteren Bauteil angeordneten Drehelementes (32) verdrehbar ist, mit einer Kupplung (90) zum lösbaren, drehschlüssigen Verbinden des einen Bauteils mit dem Drehelement (32) des weiteren Bauteils, wobei die Kupplung (90) zwei längs einer Kupplungsachse (A) ineinandergreifende Kupplungshälften (56, 76) aufweist, wobei auf jeder Kupplungshälfte (56, 76) im radialen Abstand zur Kupplung (A) zumindest zwei Zähne (58, 59, 60, 61; 78, 79) angeordnet sind, wobei sich an jeden Zahn in Umfangsrichtung zumindest eine Lücke (62, 63, 64, 65; 86, 87) anschließt, in die passend ein Zahn der anderen Kupplungshälfte eintreten kann, **dadurch gekennzeichnet, daß** jeder Zahn (58, 59, 60, 61; 78, 79) in Umfangsrichtung mit zumindest einer Abschrägung (68, 69; 81, 82) versehen ist, und daß die Zähne (58, 59, 60, 61) der einen Kupplungshälfte (56) umfänglich über Materialbrücken miteinander verbunden sind, und daß die Materialbrücken radial zurückgesetzte Aussparungen (70, 71, 72, 73) aufweisen, in die die Zähne (78, 79) der anderen Kupplungshälfte (56) derart einrücken, daß die beiden Kupplungshälften (56, 76) gegen axiales Abziehen gesperrt sind.

2. Medizinisches Instrument nach Anspruch 1, **dadurch gekennzeichnet, daß** jeder Zahn (58, 59, 60, 61; 78, 79) beidseitig einer Spitze (66, 67; 80) mit einer Abschrägung (68, 69) versehen ist.

3. Medizinisches Instrument nach Anspruch 1, **dadurch gekennzeichnet, daß** auf einer Kupplunghälfte (56) vier Zähne (58, 59, 60, 61) und auf der weiteren Kupplungshälfte (76) zwei Zähne (78, 79) vorgesehen sind.

4. Medizinisches Instrument nach einem der Ansprüche 1 bis 3, **dadurch gekennzeichnet, daß** die Abschrägungen der Zähne in einem spitzen Winkel zueinander stehen.

5. Medizinisches Instrument nach einem der Ansprüche 1 bis 3, **dadurch gekennzeichnet, daß** die Abschrägungen der Zähne in einem stumpfen Winkel zueinander stehen.

6. Medizinisches Instrument nach einem der Ansprüche 1 bis 3, **dadurch gekennzeichnet, daß** die Abschrägungen (68, 69; 81, 82) der Zähne (58, 59, 60, 61; 78, 79) in einem Winkel von etwa 90° zueinander stehen.

7. Medizinisches Instrument nach einem der Ansprüche 1 bis 6, **dadurch gekennzeichnet, daß** die Spitzen (63-66; 80) der Zähne (58, 59, 60, 61; 78, 79) scharfe Kanten aufweisen.

8. Medizinisches Instrument nach einem der Ansprüche 1 bis 6, **dadurch gekennzeichnet, daß** die Spitzen der Zähne abgerundet sind.

9. Medizinisches Instrument nach einem der Ansprüche 1 bis 8, **dadurch gekennzeichnet, daß** die Abschrägungen der Zähne nach außen gekrümmt verlaufen.

10. Medizinisches Instrument nach einem der Ansprüche 1 bis 9, **dadurch gekennzeichnet, daß** die Zähne magnetische Spitzen (63-66; 80) aufweisen, wobei die magnetischen Spitzen der einen Kupplungshälfte (56) gegenpolig zu den magnetischen Spitzen der anderen Kupplungshälfte (76) sind.

11. Medizinisches Instrument nach einem der Ansprüche 1 bis 10, **dadurch gekennzeichnet, daß** die beiden Bauteile über eine von der Kupplung (90) getrennte Verriegelung (30) gegen axiales Trennen verriegelt sind.

12. Medizinisches Instrument nach Anspruch 11, **dadurch gekennzeichnet, daß** bei einem Rohrschaftinstrument (10) vier Zähne (58, 59, 60, 61) an der Außenseite eines Rohrschaftes (14) ausgebildet sind, und daß axial davon beabstandet eine Ringnut (48) der Verriegelung (30) ausgespart ist, daß an einer Innenseite (83) des Drehelementes (82) am Handgriff (12) zwei diametral gegenüberliegende Zähne (78, 79) vorgesehen sind, und daß am Handgriff (12) ein Riegel (28) vorgesehen ist, der sperrend in die Ringnut (48) eingreift.

## Claims

1. Medical instrument that is disassemblable into several components, in particular into a handle (12) and a tubular shaft (14), one component, in particular the tubular shaft (14) being rotatable with respect to a further component, in particular the handle (12) by means of a rotary element (32) arranged on the further component; having a coupling (90) for detachable rotationally engaged connection of the one component to the rotary element (32) of the further component, the coupling (90) having two coupling halves (56, 76) which engage into one another along a coupling axis (A), wherein there is arranged on each coupling half (56, 76) at a radial spacing from the coupling axis (A) at least two teeth (58, 59, 60, 61; 78, 79); adjacent to each tooth in the circumferential direction is at least one gap (62, 63, 64, 65; 86, 87) into which a tooth of the other coupling half can enter and fit, **characterized in that** each tooth (58, 59, 60, 61; 78, 79) is equipped in the circumferential direction with at least one bevel (68, 69; 81, 82), and in that the teeth (58, 59, 60, 61) of one coupling half (56) are joined to one another circumferentially via material bridges, and the material bridges have radially set-back recesses (70, 71, 72, 73) into which the teeth (78, 79) of the other coupling half (56) engage in such a way that the two coupling halves (56, 76) are inhibited in terms of axial separation.

2. Medical instrument according to claim 1, **characterized in that** each tooth (58, 59, 60, 61, 78, 79) is equipped with a bevel (68, 69) on either side of a tip (66, 67; 80).

3. Medical instrument according to claim 1, **characterized in that** four teeth (58, 59, 60, 61) are provided on one coupling half (56) and two teeth (78, 79) on the further coupling half (76).

4. Medical instrument according to anyone of claims 1 through 3, **characterized in that** the bevels of the teeth are at an acute angle to one another.

5. Medical instrument according to anyone of claims 1 through 3, **characterized in that** the bevels of the teeth are at an oblique angle to one another.

6. Medical instrument according to anyone of claims 1 through 3, **characterized in that** the bevels (68, 69; 81, 82) of the teeth (58, 59, 60, 61; 78, 79) are at an angle of approximately 90° to one another.

7. Medical instrument according to anyone of claims 1 through 6, **characterized in that** the tips (63-66; 80) of the teeth (58, 59, 60, 61; 78, 79) have sharp edges.

8. Medical instrument according to anyone of claims 1 through 6, **characterized in that** the tips of the teeth are rounded.

9. Medical instrument according to anyone of claims 1 through 8, **characterized in that** the bevels of the teeth are curved outward in profile.

10. Medical instrument according to anyone of claims 1 through 9, **characterized in that** the teeth have magnetic tips (63-66; 80), the magnetic tips of the one coupling half (56) being opposite in polarity to the magnetic tips of the other coupling half (76).

11. Medical instrument according to anyone of claims 1 through 10, **characterized in that** the two components are locked in terms of axial separation by an interlock (30) that is separate from the coupling (90).

12. Medical instrument according to claim 11, **characterized in that** in a tubular shaft instrument (10) four teeth (58, 59, 60, 61) are configured on the exterior of the tubular shaft (14); and at an axial spacing therefrom an angular groove (48) of the interlock (30) is cut in; two diametrically opposite teeth (78, 79) are provided on an inner side (83) of the rotary element (82) on the handle (12), and a locking element (28) which engages inhibitingly into the annular groove (48) is provided on the handle (12).

## Revendications

1. Instrument médical qui peut être décomposé en plusieurs composants, notamment en une poignée (12) et une tige tubulaire (14), un composant, en particulier la tige tubulaire (14), pouvant tourner par rapport à un autre composant, en particulier la poignée (12), au moyen d'un élément tournant (32) disposé sur l'autre composant, comportant un accouplement (90), pour la liaison amovible, par rotation d'un composant avec l'élément tournant (32) de l'autre composant, l'accouplement (90) comportant deux moitiés d'accouplement (56, 76) s'engageant l'une dans l'autre le long d'un axe d'accouplement (A), sur chaque moitié d'accouplement (56, 76) étant disposées, à distance radiale de l'accouplement (A), au moins deux dents (58, 59, 60, 61 ; 78, 79), à chaque dent faisant suite, dans la direction périphérique, au moins un entredent (62, 63, 64, 65 ; 86, 87) dans lequel une dent de l'autre moitié d'accouplement peut pénétrer de manière ajustée, **caractérisé en ce que** chaque dent (58, 59, 60, 61 ; 78, 79) est pourvue dans la direction périphérique d'au moins un chanfrein (68, 69 ; 81, 82), et en ce que les dents (58, 59, 60, 61) d'une moitié d'accouplement (56) sont reliées entre elles périphériquement par des ponts de matière, et en ce que les ponts de matière présentent des évidements (70, 71, 72, 73) en retrait radialement, dans lesquelles les dents (78, 79) de l'autre moitié d'accouplement (56) engrènent de manière que les deux moitiés d'accouplement (56, 76) soient bloquées par rapport à une séparation axiale.

2. Instrument médical selon la revendication 1, **caractérisé en ce que** chaque dent (58, 59, 60, 61 ; 78, 79) est pourvue d'un chanfrein (68, 69), des deux côtés d'un sommet (66, 67 ; 80).

3. Instrument médical selon la revendication 1, **caractérisé en ce que** quatre dents (58, 59, 60, 61) sont prévues sur une moitié d'accouplement (56) et deux dents (78, 79) sur l'autre moitié d'accouplement (76).

4. Instrument médical selon l'une des revendications 1 à 3, **caractérisé en ce que** les chanfreins des dents forment entre eux un angle aigu.

5. Instrument médical selon l'une des revendications 1 à 3, **caractérisé en ce que** les chanfreins des dents forment entre eux un angle obtus.

6. Instrument médical selon l'une des revendications 1 à 3, **caractérisé en ce que** les chanfreins (68, 69 ; 81, 82) des dents (58, 59, 60, 61 ; 78, 79) forment entre eux un angle d'environ 90°.

7. Instrument médical selon l'une des revendications 1 à 6, **caractérisé en ce que** les sommets (63 à 66 ; 80) des dents (58, 59, 60, 61 ; 78, 79) présentent des arêtes vives.

8. Instrument médical selon l'une des revendications 1 à 6, **caractérisé en ce que** les sommets des dents sont arrondis.

9. Instrument médical selon l'une des revendications 1 à 8, **caractérisé en ce que** les chanfreins des dents sont courbés vers l'extérieur.

10. Instrument médical selon l'une des revendications 1 à 9, **caractérisé en ce que** les dents présentent des sommets magnétiques (63 à 66; 80), les sommets magnétiques de l'une des moitiés d'accouplement (56) étant de polarité opposée aux sommet magnétiques de l'autre moitié d'accouplement (76).

11. Instrument médical selon l'une des revendications 1 à 10, **caractérisé en ce que** les deux composants sont verrouillés contre une séparations axiale, au moyen d'un verrouillage (30) séparé de l'accouplement (90).

12. Instrument médical selon la revendication 11, **caractérisé en ce que** dans le cas d'un instrument à tige tubulaire (10), quatre dents (58, 59, 60, 61) sont formées sur le côté extérieur d'une tige tubulaire (14) et en ce qu'à distance axiale de ces dents, il est découpé une rainure annulaire (48) du verrouillage (30), en ce que sur un côté intérieur (83) de l'élément tournant (82), deux dents (78, 79) diamétralement opposées sont prévues sur la poignée (12), et en ce qu'il est prévu sur la poignée (12) un verrou (28) qui s'engage dans la rainure annulaire (48), en provoquant un blocage.
